# EUROPEAN PATENT APPLICATION

(11) **EP 2 949 662 A1**
(43) Date of publication of application: **02.12.2015**
(21) Application number: 15165607.1
(22) Date of filing: 26.06.2008
(51) Int. Cl.: C07K 14/435, G01N 33/68, C07K 14/705, C07K 14/72

(54) **CHOLINERGIC/SEROTONINERCIC CHIMERIC RECEPTOR AND USES THEREOF**

(30) Priority: 27.06.2007 US 946583 P
(62) Divisional of application: 08771975.3
(71) Applicant: AbbVie Inc., North Chicago, IL 60064 (US)
(72) Inventor: Gopalakrishnan, Murali, Libertyville, IL 60048 (US); Li, Jinhe, Long Grove, IL 60047 (US); Cassar, Steven C., Kenosha, WI 53143 (US); Malysz, John, Round Lake, IL 60073 (US); Anderson, David J., Lake Bluff, IL 60044 (US); Gubbins, Earl J., Libertyville, IL 60048 (US); Bertrand, Daniel C., 1204 Geneva (CH)
(74) Representative: Modiano, Micaela Nadia

(57) **Abstract**

The present invention describes new cholinergic/serotoninergic chimeric receptors and provides methods and compositions suitable for screening for ligands such as agonists, antagonists and allosteric modulators of α7 nicotinic acetylcholine receptors.

## Description

### FIELD AND BACKGROUND OF THE INVENTION

The present invention relates to alpha-7 nicotinic acetylcholine receptor (α7 nAChR) chimeric receptors containing one or more regions homologous to a nicotinic cholinergic receptor and a serotoninergic receptor for measuring α7 nAChR function and methods and compositions useful in the identification of α7 nAChR agonists, antagonists and allosteric modulators.

Ion channels are hydrophilic pores across the cellular membrane that open in response to stimulation to allow specific inorganic ions of appropriate size and charge to pass across the membrane. Depending on the nature of the ligand, ion channels expressed in the plasma membrane are broadly classified as voltage-gated ion channels (VGIC) or ligand-gated ion channels (LGIC) where the ligand usually is considered to be an extracellular messenger such as a neurotransmitter (Gopalakrishnan and Briggs, 2006). Specific residues in ion channel proteins also determine the specificity for the inorganic ion transported including sodium, potassium, calcium, and chloride ions.

Ligand-gated ion channels are essential in mediating communication between cells. These channels convert a chemical signal (often a neurotransmitter, as for example, acetylcholine) released by one cell into an electrical signal that propagates along a target cell membrane through specific ion influx. A variety of neurotransmitters and neurotransmitter receptors exist in the central and peripheral nervous systems. Numerous families of ligand-gated receptors have been identified and categorized by their specific ligands and on the basis of sequence identity. These include receptors specific for acetylcholine, glutamate, glycine, GABA A, and 5-HT.

nAChRs receptors, members of the cys-loop superfamily of LGIC, are widely characterized transmembrane proteins involved in the physiological responses to the neurotransmitter ACh and are distributed throughout both the central nervous system (CNS) and the peripheral nervous system (PNS). The nicotinic acetylcholine receptors (nAChRs) are multiunit proteins of neuromuscular and neuronal origins and mediate synaptic transmission between nerve and muscle and between neurons upon interaction with the neurotransmitter acetylcholine (ACh). Organizationally, nAChRs are homopentamers or heteropentamers composed of nine alpha and four beta subunits that co-assemble to form multiple subtypes of receptors that have a distinctive pharmacology. ACh is the endogenous ligand (agonist), while nicotine is a prototypical agonist that non-selectively activates all nAChRs. Functional nAChRs are widely expressed in the central nervous system and in the ganglia of the autonomic nervous system. nAChRs are involved in a range of synaptic and extra synaptic functions. In the peripheral nervous system, nAChRs mediate ganglionic neurotransmission whereas in the CNS, nicotinic cholinergic innervation mediates fast synaptic transmission and regulates processes such as transmitter release, synaptic plasticity and neuronal network integration by providing modulatory input to a range of other neurotransmitter systems. Thus, nAChR subtypes are implicated in a range of physiological and pathophysiological functions related to cognitive functions, learning and memory, reward, motor control, arousal and analgesia.

The α7 nAChR is a ligand-gated calcium channel formed by a homopentamer of α7 subunits. These receptors are expressed in several brain regions, especially localized at presynaptic and postsynaptic terminals in the hippocampus and cerebral cortex, regions critical to the synaptic plasticity underlying learning and memory. Presynaptic α7 nAChRs present on GABAergic, glutamatergic and cholinergic neurons can facilitate directly or indirectly the release of neurotransmitters such as glutamate, GABA and norepinephrine whereas postsynaptic receptors can modulate other neuronal inputs and trigger a variety of downstream signaling pathways. This facilitation of pre- and post-synaptic mechanisms by α7 nAChRs could influence synaptic plasticity, important for cognitive functions involved in attention, learning, and memory. Support for this hypothesis has emerged from preclinical studies with selective agonists, antagonists, and more recently, positive allosteric modulators (PAMs). Structurally diverse α7 nAChR agonists such as PNU-282987, SSR-180711A and AR-R17779 can improve performance in social recognition (Van Kampen, M. et. al., 2004), maze training (Levin, E. D. et. al., 1999; Arendash, G. W. et. al, 1995) and active avoidance (Arendash, G. W. et. al, 1995) models while α7 nAChR antagonists or antisense impair such performance (Bettany, J. H. et. al., 2001; Felix, R. and Levin, E. D., 1997; Curzon, P. et. al., 2006). Both agonists and PAMs, exemplified respectively by PNU-282987 and PNU-120596, have also been shown to reverse auditory gating deficits in animal models (Hajos, M. et. al., 2005; Hurst et al, 2005).

Although α7 nAChRs have significant Ca2+ permeability comparable to NMDA receptors, these receptors do not require membrane depolarization for activation, and the current responses are curtailed by rapid receptor desensitization processes (Quick, M. W., and Lester, R. A. J., 2002). The functional significance of α7 nAChRs is not only attributable to its electrogenic properties (i.e. modulation of neuronal excitability and neurotransmitter release) but also to its high Ca²⁺-permeability and association with biochemical signaling pathways. Thus, activation of α7 nAChR can result in increased intracellular Ca2+, leading to signal transduction cascades involving the activation of a variety of protein kinases and other proteins by phosphorylation. Proteins that are phosphorylated in response to α7 nAChR activation could include extracellular signal-regulated kinase 1/2 (ERK1/2) (Ren, K. et. al., 2005), cAMP response element binding protein (CREB) (Roman, J. et. al., 2004) and Akt (Shaw, S. H. et. al., 2002).

The rapid receptor desensitization (within 50-100 milliseconds) of α7 nAChRs greatly limits the development of functional assays required for measurement of channel activity. A simple and high throughput assay is critical for screening for ligands that interact with the α7 nAChR with potential for the treatment of diseases where cognitive deficits remain an underlying component.

Serotonin (5-hydroxytryptamine, or 5-HT) receptors belong to at least two superfamilies: G-protein-associated receptors and ligand-gated ion channels. The majority of 5-HT receptors couple to effector molecules through G-protein coupled receptors. However, the 5-HT₃ receptor functions as a rapidly activating ion channel and, like other LGIC family members, incorporates a nonselective cation channel in its primary structure. 5-HT₃ receptors are expressed in native central and peripheral neurons where they are thought to play important roles in sensory processing and control of autonomic reflexes (Richardson, B. P., et al., 1985). 5-HT3 channels desensitize much slower than α7 nAChR.

Therefore, a chimeric receptor prepared from the human N-terminal ligand binding domain of α7 nAChR and the pore forming C-terminal domain of the human 5-HT3 would preserve the ligand selectivity for human α7 nAChR while delay the desensitization of the receptor. The delayed desensitization would make it easier to measure the channel function of α7 nAChR. Other amino acid stretches containing different segments of the α7 nAChR could be introduced to generate additional chimeras. Such chimeric receptors would be particularly useful for functional screening and identifying novel α7 nAChR agonists, modulators and antagonists.

### BRIEF DESCRIPTION OF THE FIGURES

Figure 1. Schematic representation of cholinergic (α7)/serotoninergic (5HT₃) chimeras 1-8.
Figure 2. Illustration of expression of cholinergic (α7)/ serotoninergic (5HT₃) chimeras by electrophysiology (two electrode voltage clamp).
Figure 3. HEK-293 cells stably expressing chimera 1 and 2 express functional currents with distinct properties.
Figure 4. Effects of α7 agonists in HEK-293 cells stably expressing chimera 2.
Figure 5. Effects of genistein on Ach evoked responses in chimera 2 expressing cells and in wild type.
Figure 6. Effects of modulators 5-HI, genistein and NS1738 on chimera 2.
Figure 7. Genistein potentiation of chimera 2 and not of chimera 1.
Figure 8. Effects of PAMs NS1738NS1738 and PNU-120596 in chimera 1 on responses induced by Ach.
Table 1. Summary of α7 agonist effects in chimera 1 and 2 expressing *Xenopus leavis* or HEK-293 cells stably expressing chimeras studied using electrophysiology (POETs), radioligand binding, and FLIPR-FMP.

### SEQUENCE LISTING

SEQ ID NO. 1: polynucleotide human-human chimera 1
SEQ ID NO. 2: polynucleotide human-human chimera 2
SEQ ID NO. 3: polynucleotide human-human chimera 3
SEQ ID NO. 4: polynucleotide human-human chimera 4
SEQ ID NO. 5: polynucleotide human-human chimera 5
SEQ ID NO. 6: polynucleotide human-human chimera 6
SEQ ID NO. 7: polynucleotide human-human chimera 7
SEQ ID NO. 8: polynucleotide human-human chimera 8
SEQ ID NO. 9: polypeptide human-human chimera 1
SEQ ID NO. 10: polypeptide human-human chimera 2
SEQ ID NO. 11: polypeptide human-human chimera 3
SEQ ID NO. 12: polypeptide human-human chimera 4
SEQ ID NO. 13: polypeptide human-human chimera 5
SEQ ID NO. 14: polypeptide human-human chimera 6
SEQ ID NO. 15: polypeptide human-human chimera 7
SEQ ID NO. 16: polypeptide human-human chimera 8

### DETAILED DESCRIPTION OF THE INVENTION

The present invention discloses fully human α7 nAChR-5HT3 chimeric receptors and an easy way to measure the channel function by delaying the desensitization, which in turn provides for a more efficient high throughput assay. Incorporation of additional amino acid stretches such as the M2-M3 segment of the α7 nAChR confers novel screening opportunities, particularly for allosteric modulators.

The principal embodiment of the present invention is a recombinant nucleic acid encoding a fully human amino acid sequence of a cholinergic/serotoninergic chimeric receptor. A preferred embodiment of said recombinant nucleic acid comprises an amino acid sequence of the fully human cholinergic/serotoninergic chimeric receptor comprising an amino acid extracellular domain with the sequence of a human neuronal nicotinic cholinergic subunit receptor, and an amino acid intracellular domain with the sequence of a human serotonin receptor. In another embodiment of the present invention the fully human cholinergic/serotoninergic chimeric receptor amino acid sequence comprises an amino acid extracellular domain with the sequence of a human neuronal nicotinic cholinergic subunit receptor, an amino acid intracellular domain with the sequence of a human serotonin receptor, and a four-transmembrane domain with an amino acid sequence of a human serotonin receptor.

A more preferred embodiment of the present invention comprises the encoded fully human cholinergic/serotoninergic chimeric receptor amino acid sequence, in which the human neuronal nicotinic cholinergic subunit is an α7 subunit and the human serotonin receptor is a 5HT₃ receptor.

Another embodiment of the present invention comprises the fully human cholinergic/serotoninergic chimeric receptor amino acid sequence, in which part of the sequence of the transmembrane domain is from a human neuronal nicotinic cholinergic subunit receptor, in which the N-terminal extracellular domain is from human serotonin receptor is a 5HT₃ receptor, and in which the transmembrane domain is from a human neuronal nicotinic cholinergic subunit receptor.

It is intended that the nucleic acid sequence of the present invention can be selected from the group consisting of SEQ.ID.NO:1, SEQ.ID.NO:2, SEQ.ID.NO:3, SEQ.ID.NO:4, SEQ.ID.NO:5, SEQ.ID.NO:6, SEQ.ID.NO:7, and SEQ.ID.NO:8. It is also intended that the amino acid sequence encoded by any of said nucleic acid sequences is selected from the group consisting of SEQ.ID.NO:9, SEQ,ID.NO:10, SEQ.ID.NO:11, SEQ.IDNO:12, SEQ.ID.NO:13, SEQ.ID.NO:14, SEQ.ID.NO:15, and SEQ.ID.NO:16.

Another embodiment of the present invention comprises a vector containing any of the recombinant nucleic acid sequences of the present invention. It is intended that the vector is operable linked to control sequences recognized by a host cell transformed with the vector.

Another embodiment of the present invention comprises a host cell comprising the vector of the present invention; it is intended that the host cell is a cell line derived from mammalian cells, primary mammalian cell cultures, or oocytes.

Another embodiment of the present invention comprises a fully human cholinergic/serotoninergic chimeric receptor encoded by the recombinant nucleic acid sequence of the present invention. It is intended that the present invention also includes a method of manufacturing the chimeric receptor of the invention, comprising a cholinergic/serotoninergic chimeric receptor with one or more regions of a human neuronal nicotinic receptor subunit and a human serotonin receptor with the vector of the invention.

Another embodiment of the present invention includes a composition comprising a cholinergic/serotoninergic chimeric receptor comprising one or more regions of a human neuronal nicotinic receptor subunit and a human serotonin receptor, preferably wherein the composition comprises any of the amino acid sequences described in the present invention.

Another embodiment includes a method of screening for compounds that bind to a region of the fully human cholinergic/serotoninergic chimeric receptor of the present invention. Said region is selected from the N-terminal domain, C-terminal domain and the extracellular loop between TM2-TM3, to modulate the activity of a neuronal nicotinic receptor. The screening method of the present invention is selected from binding or activity-based assays. Said assays can be used to determining whether the test compound binds or modulates the chimeric receptor of the present invention, wherein the binding or modulation is indicative that the test compound binds or modulates the neuronal nicotinic receptor.

A preferred embodiment of the present invention comprises a method of screening for a compound that binds or modulates the activity of a neuronal nicotinic receptor, comprising introducing a host cell expressing the chimeric receptor of the present invention into an acceptable medium, and monitoring an effect in said host cell indicative of binding or modulation of the test compound with the chimeric receptor, wherein the binding or modulation is indicative that the test compound binds or modulates the neuronal nicotinic receptor.

Another embodiment of the present invention is a kit comprising a host cell transformed or transfected with an expression vector comprising a nucleic acid sequence encoding a chimeric receptor of the present invention.

It is intended that any of the embodiments described herein can be modified in various obvious respects by the skilled in the art, and that all of the obvious modifications are included in the present invention.

A chimeric receptor prepared from the human N-terminal ligand binding domain of α7 nAChR and the pore forming C-terminal domain of the human 5-HT3 would preserve the ligand selectivity for human α7 nAChR while delaying the desensitization of the receptor. The delayed desensitization would make it easier to measure the channel function of α7 nAChR. The chimeras of the present invention that host the N-terminal fragment along with the extracellular TMII-III loop corresponding to the α7 nAChR sequence are particularly useful for functional screening and identifying novel α7 nAChR ligands, i.e. agonists, modulators and antagonists. In addition, the human-human chimeric receptors described in the present application would be expected to better preserve the nature of human α7 nAChR as compared to human-rat chimera (Hurst et al, 2005).

The α7 nAChR-5-HT3 chimeric receptors of the present invention is also useful for α7 nAChR ligand binding assays. Ligand binding can be measured using either whole cells or membrane preparations but both kinds of assays are cumbersome. Whole cell assays are usually low throughput, while the assays using isolated membranes from animal brains typically require extensive manipulation and washing to obtain a favorable signal to noise ratio. A binding assay using cell membranes from HEK-293 cells stably transfected with α7 nAChR-5HT3 chimeric receptors of the present invention that show similar binding properties to that of wild type α7 nAChR, would be extremely useful for high throughput drug screening.

Positive allosteric modulators (PAMs) have, in general, been shown not to affect α7 nAChR channel function by themselves, but can selectively enhance the effect of α7 nAChR agonists. Two types of PAMs have been described: PAM I that enhances amplitude of inward currents only (Zwart R. et. al., 2002) and PAM II that delays desensitization of the receptor and enhancing amplitude of inward currents (Hurst et al, 2005, Grønlien et al, 2007). PAM II type has been shown to enhance the acetylcholine-evoked inward currents in hippocampal interneurons on brain slice and improved the auditory gating deficit when systemically administrated to rats, suggesting that PAM II may be used as a new class of molecule that enhances α7 nAChR function and thus has the potential to treat psychiatric and neurological disorders. The binding site of PAMI/II on α7 nAChR and mechanism of their action remain unclear. These fundamental questions could be answered by using α7 nAChR-5HT3 chimeric receptors with various replacements of domains of 5-HT3 with those of α7 nAChR. More importantly, the α7 nAChR-5HT3 chimeric receptors can also be used to screen for both novel α7 agonists and positive allosteric modulators.

### (I) Definitions.

The following is a list of some of the definitions used in the present disclosure. These definitions are to be understood in light of the entire disclosure provided herein.

By "ligand" as used herein has its general meaning in the art, and refers to a natural or synthetic compound that has the capability to bind to a receptor and mediate, prevent or modify a biological effect.

By "agonist" as used herein has its general meaning in the art, and refers to a compound natural or not which has the capability to activate a receptor.

By "antagonist" as used herein has its general meaning in the art, and refers to a compound natural or not which has the capability to inhibit the activation of a receptor.

By "positive allosteric modulator" as used herein has its general meaning in the art, and refers to a compound natural or not which has the capability to enhance the effects of an agonist, endogenous or exogenously applied, and can interaction with sites on the receptor that are topographically distinct from the site for agonists (orthosteric sites).

By "selective", a compound that is selective is a compound able to activate or inhibit the activation of a specific receptor and not any other receptor. As used herein, selective or selectivity is used in reference to the nAChR.

By "desensitization" as used herein has its general meaning in the art, and refers to a process in vitro or in vivo in which persistent exposure of receptors to an ligand results in the eventual loss or diminution of receptor-activated responses.

### (II) Chimeric Receptors

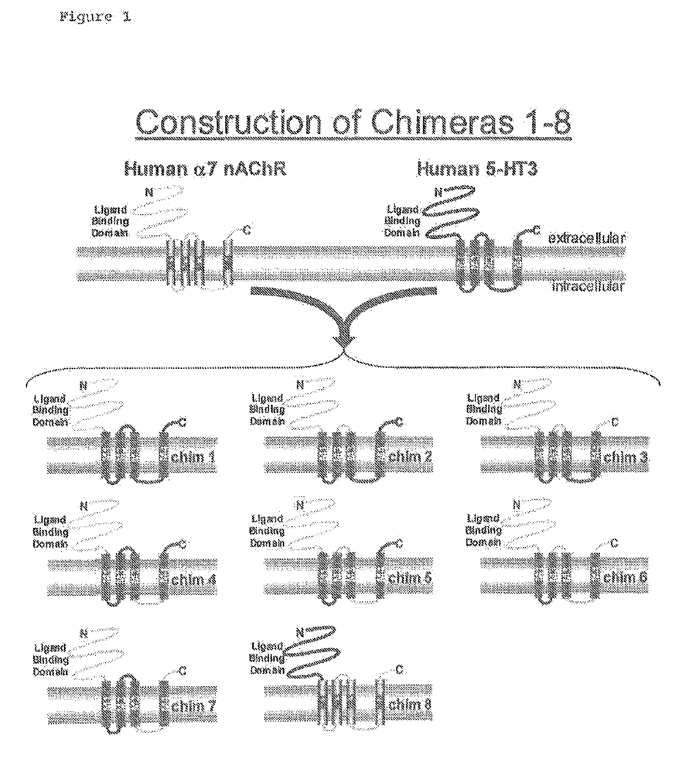

As indicated above, the present invention describes chimeric receptors that include human N-terminal ligand binding domain of α7 nAChR and the pore forming C-terminal domain of the human 5-HT3. Transmembrane regions, intracellular and extracellular loops, are also varied to obtain the chimeras of the present invention. Schematic representation of cholinergic (α7)/serotoninergic (5HT₃) chimeras 1-8, native α7 and 5HT3 constructs, are shown in Figure 1.
Chimera 1: Chimera 1 has the ligand-binding domain of α7 nAChR and the transmembrane/pore forming region of 5-HT3. Using PCR, coding sequence for the N-terminal 224 amino acids of human α7 nicotinic receptor (α7 nAChR, protein AAA83561) and that for the C-terminal 242 amino acids of human 5-hydroxytryptamine type-3 (5-HT3) serotonin receptor (protein AAP35868) were amplified with overlapping ends. Recombinant PCR using these two overlapping fragments yielded the open reading frame of Chimera 1. Primers used to generate the α7 nAChR portion of this chimera were (5' to 3¹) GCCGCCATGCGCTGCTCGCCGGGAGGCGTCT (A7F-forward) and AGGCTGACCACATAGAAGAGTGGCCTACGTCGGATGACCACTGTGAAGGTGACATCG (Chi1R-reverse). Primers used to generate the 5-HT3 portion of this chimera were (5' to 3') GTCAAGCGTACTGCCAGATGGACCAGA (5HT3R-reverse) and CGATGTCACCTTCACAGTGGTCATCCGACGTAGGCCACTCTTCTATGTGGTCAGCCT (Chi1F-forward). The primers listed in these methods were manufactured and HPLC purified by Sigma Genosys. PCR was performed in a Stratagene Robocycler using 10ng each template, 0.4µM each primer with Invitrogen Platinum^{®} *Taq* DNA Polymerase High Fidelity following Invitrogen's protocol. Recombinant PCR used 1µl of amplicon directly from each of the two reactions along with 0.4µM each of primers A7F and 5HT3R. All else are equal to the primary PCR. The recombinant PCR product was cloned into the expression vector pcDNA3.1 using Invitrogen's pcDNA3.1 TOPO TA cloning kit and transformed into DH5 alpha Max Efficiency Chemically Competent Bacteria from Invitrogen following the protocol. Clones were selected on plates containing LB agar medium and 100µg/ml ampicillin. The sequence of the inserted DNA was verified.
Chimera 2: Chimera 2 has the same amino acid composition as Chimera 1 except that 10 amino acids between transmembrane spanning (TM) region 2 and TM3 have been changed to be amino acids 280-289 of α7 nAChR (AEIMPATSDS) instead of amino acids 298-307 of 5-HT3 (SDTLPATAIG). This was accomplished through PCR amplifying two fragments that flank the region of interest, overlap each other with codons for the desired α7 nAChR sequence, and extend to unique restriction enzyme sites for EcoRI and Bsu36I that flank the region of interest. Recombinant PCR using these two fragments produced a single amplicon to be digested with the aforementioned restriction enzymes and cloned into analogous sites of Chimera 1. Primers used to generate the 5' portion of this amplicon were (5' to 3') CACACTAACGTGTTGGTGAATTCTT (A7.ECORIF-forward) and TCGGATGTTGCGGGCATGATCTCAGCAACGATGATCAGGAAGACCGAGTA (Chi2R-reverse). Primers used to generate the 3' portion of this amplicon were (5' to 3') GAAGTTGACTGCTCCCTCAGGCAA (5HT.BSUR-reverse) and ATCATGCCCGCAACATCCGATTCGACTCCTCTCATTGGTGTCTAC (Chi2F-forward). PCR was performed as described above except that Chimera 1 plasmid was used as template in each of the two reactions. Recombinant PCR used 1µl of amplicon directly from each of the two reactions along with 0.4µM each of primers A7.ECORIF and 5HT.BSUR.The product from the recombinant PCR was purified using Qiagen's Qiaquick Purification kit following the protocol. EcoRI and Bsu36I from New England Biolabs were used to digest approximately 5µg of the purified PCR product in NEBuffer 3 for 2 hours at 37°C. This digestion product (insert) was then purified using the Qiaquick method. These same restriction enzyme conditions were used to digest 1µg Chimera 1 plasmid. The Chimera 1 plasmid digestion product was electrophoresed in 0.8% agarose and the large band was purified from the small EcoRI-Bsu36I fragment by gel purification. The purified insert and plasmid were then ligated using NEB Quick Ligase following the protocol and transformed into DH5 alpha Max Efficiency Chemically Competent Bacteria from Invitrogen. Clones were selected on plates containing LB agar medium and 100µg/ml ampicillin. The sequence of the inserted DNA was verified.
Chimera 3: Chimera 3 has the same amino acid composition as Chimera 2 except that the last 3 amino acids (originally 5-HT3 amino acids 482-484, QYA) have been replaced by the 9 most C-terminal amino acids of α7 nAChR (VEAVSKDFA). This was accomplished by manufacturing the replacement sequence encoding these 9 amino acids with flanking restriction enzyme sites for NheI and ApaI and then cloning this piece into the analogous sites of Chimera 2. Primers used to manufacture the replacement sequence (5' to 3') were TATTCCACATTTACCTGCTAGCGGTGCTGGCCTACAGCATCACCCTGGTTATGCTCTG5 (HT .NHEIF-forward) and GGGCCCTCACGCAAAGTCTTTGGACACGGCCTCCACCCAGATGGACCAGAGCATAACCAGGG TGA (A7.CTAILR-reverse). These primers anneal to one another and may be extended through PCR to manufacture the desired insert. PCR was performed as described above except that no template was added to the reaction; the primers alone acted as template. Approximately 5µg of the product from this reaction was purified using Qiagen's Qiaquick Purification Kit following the protocol and digested with NheI and ApaI from New England Biolabs in NEBuffer 4 for 2 hours at 37°C. Chimera 2 plasmid (1µg) was digested similarly. Agarose electrophoresis using 0.8% agarose was used to purify the manufactured insert from its cleaved ends and also to purify the Chimera 2 plasmid from the small NheI-ApaI fragment. The prepared insert was then ligated to the prepared Chimera 2 plasmid using NEB Quick Ligase following the protocol and transformed into DH5 alpha Max Efficiency Chemically Competent Bacteria from Invitrogen. Clones were selected on plates containing LB agar medium and 100µg/ml ampicillin. The sequence of the inserted DNA was verified.
Chimera 4: Chimera 4 has the same amino acid composition as Chimera 1 except that the loop between transmembrane-3 portion(TM3) and transmembrane-4 portion (TM4) of 5HT-3 have been replaced with that of α7 nAChR. This was accomplished by combination of three fragments.
   (1) The ligand binding domain to TM3 fragments: This fragment contains the coding sequences of the human α7 nAChR ligand binding domain starting at the unique EcoRI site upstream the α7 nAChR ligand binding domain, through 5HT-3 TM3. It was generated by PCR from Chimera 1 using the following primers (5'-3') CACATTCCACACTAACGTGTTGGTGAA (A7-R1-5p-5') and ATGCCGTCTCCTCTCGGCCAAACTTATCACC (5HT3-M3-3p-3') that included a terminal BsmBI restriction enzyme site, underlined, and a 1-base silent mutation, in bold, which eliminates an existing BsmBI site. The PCR products were purified, digested with BsmBI and EcoRI, and then again purified.
   (2) TM3-TM4 fragment: This fragment contains the coding sequences of the α7 nAChR TM3-TM4 cytoplasmic loop and was generated by PCR from a cDNA clone of the human α7 nAChR receptor. Primers used to generate the "TM3-TM4" fragment were (5' to 3') were ATGCCGTCTCCGAGACCGTGATCGTGCTGCAG (A7-M3-5p-5¹) that included a terminal BsmBI restriction enzyme site, underlined; and CATGCTAGCAGGTAAATGTGGAATAGCAGCTTGTCCACCACACAGGCGG (A7-M4-3p-3') that included the 5HT3R TM4 from its beginning through its internal NheI site, underlined). The PCR product was purified, digested with BsmBI and NheI, and then purified again.
   (3) TM4 to EcoRI fragment: this fragment contains the 5HT-3 TM4, followed by 5HT-3 C-terminal, through the unique EcoRI site upstream α7 nAChR ligand binding domain. It was generated by digestion of the Chimeras 1 with EcoRI and NheI, followed by treatment with calf intestinal alkaline phosphatase and purification by gel electrophoresis.
   These three DNA fragments were ligated together with DNA Ligase. The ligations were then transformed into DH5 alpha Max Efficiency Chemically Competent Bacteria from Invitrogen. Clones were selected on plates containing LB agar medium and 100µg/ml ampicillin. The sequence of the inserted DNA was verified.
Chimera 5: Chimera 5 has the same amino acid composition as Chimera 2 except that the loop between TM3 and TM4 of 5HT-3 has been replaced with that of α7 nAChR. This was accomplished by combination of three fragments.
   (1) The ligand binding domain to TM3 fragment: This fragment contains the coding sequences of the human α7 nAChR ligand binding domain starting at the unique EcoRI site upstream the α7 nAChR ligand binding domain, through 5HT-3 TM3, in which the loop between TM2 and TM3 was from α7 nAChR. It was generated by PCR from Chimera 2 using the following primers: CACATTCCACACTAACGTGTTGGTGAA (A7-R1-5p-5') and ATGCCGTCTCCTCTCGGCCAAACTTATCACC (5HT3-M3-3p-3') that included a terminal BsmBI restriction enzyme site, underlined, and a 1-base silent mutation, in bold, which eliminates an existing BsmBI site. The PCR products were purified, digested with BsmBI and EcoRI, and then again purified.
   (2) TM3-TM4 fragment: This fragment contains the coding sequences of the α7 nAChR TM3-TM4 cytoplasmic loop and was generated by PCR from a cDNA clone of the human α7 nAChR receptor. Primers used to generate the "TM3-TM4" fragment were (5' to 3') ATGCCGTCTCCGAGACCGTGATCGTGCTGCAG (A7-M3-5p-5') that included a terminal BsmBI restriction enzyme site (underlined) and CATGCTAGCAGGTAAATGTGGAATAGCAGCTTGTCCACCACACAGGCGG (A7-M4-3p-3') that included the 5HT3R TM4 from its beginning through its internal NheI site (underlined). The PCR product was purified, digested with BsmBI and NheI, and then purified again.
   (3) TM4 to EcoRI fragment: this fragment contains the 5HT-3 TM4, followed by 5HT-3 C-terminal, through the unique EcoRI site upstream α7 nAChR ligand binding domain. It was generated by digestion of the Chimeras 2 with EcoRI and NheI, followed by treatment with calf intestinal alkaline phosphatase and purification by gel electrophoresis.
   These three DNA fragments were ligated together with DNA Ligase. The ligations were then transformed into DH5 alpha Max Efficiency Chemically Competent Bacteria from Invitrogen. Clones were selected on plates containing LB agar medium and 100µg/ml ampicillin. The sequence of the inserted DNA was verified.
Chimers 6: Chimera 6 has the same amino acid composition as Chimera 3 except that the loop between TM3 and TM4 of 5HT-3 has been replaced with that of α7 nAChR. This was accomplished by combination of three fragments.
   (1) The ligand binding domain to TM3 fragment: This fragment contains the coding sequences of the human α7 nAChR ligand binding domain starting at the unique EcoRI site upstream the α7 nAChR ligand binding domain, through 5HT-3 TM3, in which the loop between TM2 and TM3 was from α7 nAChR. It was generated by PCR from Chimera 3 using the following primers: CACATTCCACACTAACGTGTTGGTGAA (A7-R1-5p-5') and ATGCCGTCTCCTCTCGGCCAAACTTATCACC (5HT3-M3-3p-3') that included a terminal BsmBI restriction enzyme site, underlined, and a 1-base silent mutation, in bold, which eliminates an existing BsmBI site. The PCR products were purified, digested with BsmBI and EcoRI, and then again purified.
   (2) TM3-TM4 fragment: This fragment contains the coding sequences of the α7 nAChR TM3-TM4 cytoplasmic loop and was generated by PCR from a cDNA clone of the human α7 nAChR receptor. Primers used to generate the "TM3-TM4" fragment were (5' to 3') ATGCCGTCTCCGAGACCGTGATCGTGCTGCAG (A7-M3-5p-5') that included a terminal BsmBI restriction enzyme site (underlined) and CATGCTAGCAGGTAAATGTGGAATAGCAGCTTGTCCACCACACAGGCGG (A7-M4-3p-3') that included the 5HT3R TM4 from its beginning through its internal NheI site (underlined). The PCR product was purified, digested with BsmBI and NheI, and then purified again.
   (3) TM4 to EcoRI fragment: this fragment contains the 5HT-3 TM4, followed by α7 nAChR C-terminal, through the unique EcoRI site upstream α7 nAChR ligand binding domain. It was generated by digestion of the Chimeras 3 with EcoRI and NheI, followed by treatment with calf intestinal alkaline phosphatase and purification by gel electrophoresis.
   These three DNA fragments were ligated together with DNA Ligase. The ligations were then transformed into DH5 alpha Max Efficiency Chemically Competent Bacteria from Invitrogen. Clones were selected on plates containing LB agar medium and 100µg/ml ampicillin. The sequence of the inserted DNA was verified.
Chimera 7: Chimera 7 has the same amino acid composition as Chimera 4 except that the 5HT-3 C-terminal has been replaced with the α7 nAChR C-terminal. This was accomplished by combination of three fragments.
   (1) The ligand binding domain to TM3 fragment: This fragment contains the coding sequences of the human α7 nAChR ligand binding domain starting at the unique EcoRI site upstream the α7 nAChR ligand binding domain, through 5HT-3 TM3. It was generated by PCR from Chimera 1 using the following primers: CACATTCCACACTAACGTGTTGGTGAA (A7-R1-5p-5') and ATGCCGTCTCCTCTCGGCCAAACTTATCACC (5HT3-M3-3p-3') that included a terminal BsmBI restriction enzyme site (underlined) and a 1-base silent mutation (in bold) which eliminates an existing BsmBI site. The PCR products were purified, digested with BsmBI and EcoRI, and then again purified.
   (2) TM3-TM4 fragment: This fragment contains the coding sequences of the α7 nAChR TM3-TM4 cytoplasmic loop and was generated by PCR from a cDNA clone of the human α7 nAChR receptor. Primers used to generate the "TM3-TM4" fragment were (5' to 3') ATGCCGTCTCCGAGACCGTGATCGTGCTGCAG (A7-M3-5p-5') that included a terminal BsmBI restriction enzyme site (underlined) and CATGCTAGCAGGTAAATGTGGAATAGCAGCTTGTCCACCACACAGGCGG (A7-M4-3p-3') that included the 5HT3R TM4 from its beginning through its internal NheI site (underlined). The PCR product was purified, digested with BsmBI and NheI, and then purified again.
   (3) TM4 to EcoRI fragment: this fragment contains the 5HT-3 TM4, followed by α7 nAChR C-terminal, through the unique EcoRI site upstream α7 nAChR ligand binding domain. It was generated by digestion of the Chimeras 3 with EcoRI and NheI, followed by treatment with calf intestinal alkaline phosphatase and purification by gel electrophoresis.
   These three DNA fragments were ligated together with DNA Ligase. The ligations were then transformed into DH5 alpha Max Efficiency Chemically Competent Bacteria from Invitrogen. Clones were selected on plates containing LB agar medium and 100µg/ml ampicillin. The sequence of the inserted DNA was verified.
Chimera 8 (Reverse Chimera): Chimera 8, as the reverse form of chimera 1, has the ligand-binding domain of 5-HT3 and the transmembrane/pore-forming region of α7 nAChR. Using PCR, coding sequence for the 5HT-3 N-terminal and the α7 nAChR C-terminal were amplified with overlapping ends. Recombinant PCR using these two overlapping fragments yielded the open reading frame of Chimera 8. Primers used to generate the 5-HT3 portion of this chimera were (5' to 3') GCCGCCATGCTTGGAAAGCTCGCTATGCT (5HT3F-forward) and AGCGTCCTGCGGCGCATGGTCACATAGAACTTCATTTCTG (RChi1R-reverse). Primers used to generate the α7 nAChR portion of this chimera were (5' to 3') GTTACGCAAAGTCTTTGGACACGGC (A7R-reverse) and CAGAAATGAAGTTCTATGTGACCATGCGCCGCAGGACGCT (RChi1F-froward). PCR was performed in a Stratagene Robocycler using 10ng each template, 0.4µM each primer with Invitrogen Platinum^{®} Tag DNA Polymerase High Fidelity following Invitrogen's protocol. Recombinant PCR used 1µl of amplicon directly from each of the two reactions along with 0.4µM each of primers 5HT3F and A7R and was carried out identically to that for the generation of the Chimera 1 recombinant product. The recombinant PCR product was cloned into the expression vector pcDNA3.1 using Invitrogen's pcDNA3.1 TOPO TA cloning kit and transformed into DH5 alpha Max Efficiency Chemically Competent Bacteria from Invitrogen following the protocol. Clones were selected on plates containing LB agar medium and 100µg/ml ampicillin. The sequence of the inserted DNA of was verified.

### (III) Techniques

### (1) ELECTROPHYSIOLOGY

*Xenopus laevis* oocytes were prepared and injected as previously described {Eisele, 1993 #2; Krause, 1998 #4}. Briefly, ovaries were harvested from female Xenopus. Isolation of the oocytes was obtained by enzymatic dissociation using collagenase type I in a medium deprived of calcium and by gentle mechanical agitation for approximately 3 hours. Oocytes stage 5-6 were manually selected on the next day and injected into the nucleus with 2 ng plasmid containing the cDNA of interest. Oocytes were then placed in a 96 well microtiter plate in Barth solution and used for electrophysiological investigation two to five days later. All recordings were performed at 18 °C and cells were superfused with OR2 medium containing in mM : NaCl 82.5, KCl 2.5, HEPES 5, CaCl₂.2H₂O 2.5, MgCl₂.6H₂O 1, pH 7.4, and 0.5 µM atropine was added to prevent possible activation of endogenous muscarinic receptors. Unless indicated cells were held at -100 mV using a two electrode voltage clamp apparatus connected to a Geneclamp amplifier (Molecular Devices). Data were captured and analyzed using data acquisition and analysis software. Concentration-response curves were fit using the empirical Hill equation: Y=1 / 1+(EC₅₀/x)^n*_{H}* where: y = the fraction of remaining current, EC₅₀ = concentration of half inhibition, *n_{H}* = the apparent cooperativity, x = agonist concentration. Values indicated throughout the text are given with their respective standard error of the mean (SEM). For statistical analysis we used the unpaired, two-tailed Student's T test using either excel (Microsoft) or Matlab (Mathworks Inc.).

### (2) MEMBRANE POTENTIAL MEASUREMENT

HEK-293 cells stably expressing human α7 nAChR-5HT3 chimeric receptors were grown to confluence in 162-175 cm² tissue culture flasks in Dulbecco's Modified Eagle Media (DMEM) supplemented with 10% fetal bovine serum (FBS) and 0.6 mg/ml G-418. The cells were then dissociated using cell dissociation buffer and resuspended in the growth medium. Cells were plated at 100 ul of cell suspension (∼ 60,000 - 80,000 cells/well) into 96-well black plates (poly-D-lysine precoated) with clear bottom and maintained for 24-48 hrs in a tissue culture incubator at 37°C under an atmosphere of 5% CO₂: 95% air. On the day of testing, responses were measured using Fast Membrane Potential (FMP) dye (Molecular Devices) according to manufacturer's instructions. Briefly, a stock solution of the dye was prepared by dissolving each vial supplied by the vendor in low Ca²⁺ and low Mg²⁺ Hank's balanced salt solution buffer (HBSS) containing 10 mM HEPES and 0.5 uM atropine. The low Ca²⁺ and Mg²⁺ HBSS buffer was obtained by adding 0.1 mM CaCl2 and 0.1 mM MgCl2 to Ca²⁺ and Mg²⁺ free HBSS. Instead of Ca²⁺ and Mg²⁺ free HBSS, Ca²⁺ and Mg²⁺ free PBS can also be used. The dye stock solution was diluted 1:10 with the same buffer before use. The growth media was removed from the cells. The cells were loaded with 100 ul of the dye per well and incubated at room temperature for up to 1 hr. Fluorescence measurements were read simultaneously from all the wells by a Fluorometic Imaging Plate Reader (FLIPR) at an excitation wavelength of 480 nm and by using an emission filter provided by Molecular Devices specifically for the fluorescence membrane potential (FMP). Depending on the purpose of experiments either a single addition or double addition protocol was used. In a single addition (agonist) protocol, the basal fluorescence was measured for 10 sec and 50 ul of compounds (3-fold higher concentration) was added, and responses measured for up to 10 min. In the double addition (modulator) protocol, basal fluorescence was measured for 10 sec then 50 ul (3-fold higher concentration) of test compounds were added in the first addition for 5 - 10 min followed by 50 ul of the second compound addition (4-fold higher concentration). The double addition protocol can be used to measure antagonist or positive allosteric modulator activity when the second addition utilizes submaximum concentration of an agonist. Data were normalized to maximal responses of a reference α7 nAChR agonist (100 uM acetylcholine or 1 uM NS6784) and plotted as a function of concentration in agonist experiments or to submaximum response of the reference agonist (60-120 nM NS6784),

### (3) RADIOLIGAND BINDING

[³H]-A585539, also known as ([³H]-(S,S)-2,2-dimethyl-5-(6-phenyl-pyridazin-3-yl)-5-aza-2-azonia-bicyclo[2.2.1] heptane iodide) or [³H]-DPPB (U.S. patent application number 20070072892A1), binding to α7 nAChR-5HT3 chimeric receptors was determined using cellular membranes. Adherent cells were scraped from tissue culture flasks using Dulbecco's PBS with 0.1 mM PMSF. The cells were centrifuged at 500 X g for 10 min and the pellets were homogenized with a Polytron at a setting of 7 for 20 sec in 30 volumes of BSS-Tris buffer (120 mM NaCl, 5 mM KCl, 2 mM CaCl₂, 2 mM MgCl₂, and 50 mM Tris-Cl, pH 7.4, 4°C). After centrifugation at 500 x g for 10 min, the resultant supernatant was centrifuged at 40,000 x g for 15 min. The membrane pellets were resuspended in BSS to result in 2-5 mg protein per aliquot. Maximal binding levels (B_{MAX}) and dissociation constants (K_{D}) were determined using 8-16 concentrations from 0.05 to 5 nM of [³H]-A585539 (62.8 Ci/mmol; R46V, Abbott Labs). Samples were incubated in a final volume of 500 µl for 75 min at 4°C in quadruplicate. Non-specific binding was determined in the presence of 10 µM (-)nicotine in duplicate. Bound radioactivity was collected on Millipore MultiScreen® harvest plates FB presoaked with 0.3% PEI using a PerkinElmer cell harvester, washed with 2.5 ml ice-cold buffer, and radioactivity was determined using a PerkinElmer TopCount® microplate beta counter. K_{D} and B_{MAX} values were determined from nonlinear regression analysis of untransformed data using GraphPad Prism®. For displacement curves, seven log-dilution concentrations of test compounds containing 2-5 µg of protein, and 0.5 nM [³H]-A585539 (62.8 Ci/mmol; R46V, Abbott Labs) were incubated in a final volume of 500 µl for 75 minutes at 4°C in duplicate. Non-specific binding was determined in the presence of 10µM methyllycaconitine. IC₅₀ values were determined by nonlinear regression in Microsoft® Excel or Assay Explorer. Kᵢ values were calculated from the IC₅₀s using the Cheng-Prusoff equation, where Kᵢ = IC₅₀/(1+[Ligand]/K_{D}).

### (IV) Examples

### Example 1. EXPRESSION OF CHIMERAS AND RESPONSES TO α7 nAChR AGONISTS

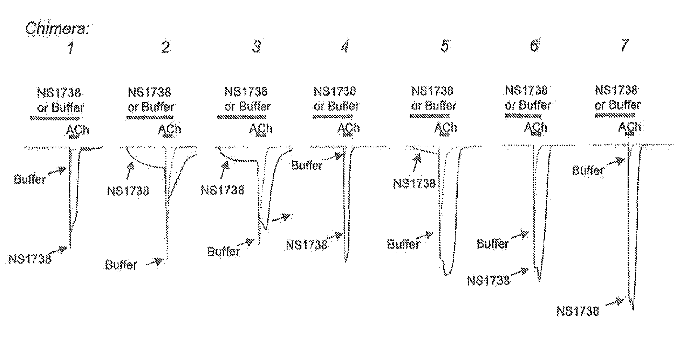

All engineered chimeras contain the α7 encoded N-terminal extracellular region, which contains the agonist binding sites. Therefore, α7 agonists, but not 5-HT3A agonists, should activate these channels. All α7-5HT3 chimeras were screened for functional expression by injecting the cDNA in *Xenopus laevis* oocytes. Figure 2 shows all 7 chimeras expressed in *Xenopus* oocytes were activated by acetylcholine (Ach) by electrophysiology (two electrode voltage clamp). As demonstrated in the figure, Ach activated currents in all chimeras. Figure 2 also shows that NS1738, a positive allosteric modulator NS1738 can differentially potentiate various chimeras activated by the endogenous agonist, acetylcholine. Secondly, unlike at the wild type α7 nAChRs, NS1738 alone generally activated current responses when the α7 encoded sequence for extracellular TMII-III loop was present.

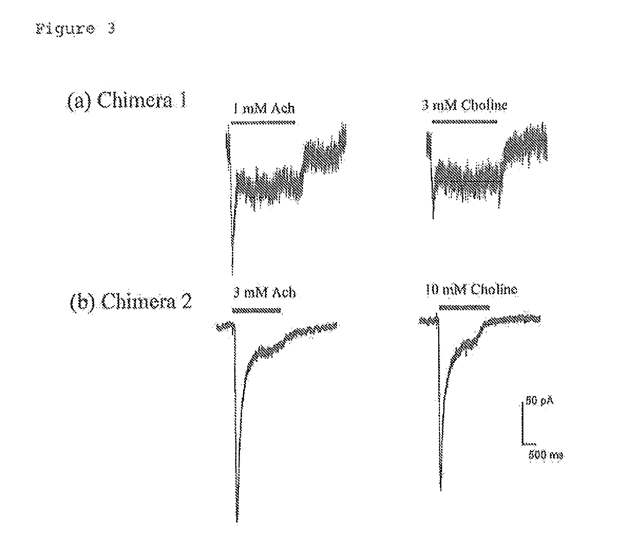

The α7 nAChR-like channel function of the chimeras was confirmed by currents evoked by ACh and choline in HEK-293 cells stably expressing chimera 1 and 2. Figure 3 (a) and (b) show representative currents evoked by Ach and choline, as indicated by horizontal bars, in HEK-293-chimera 1 and HEK-293-chimera 2 cells, respectively. Responses were measured using the patch clamp technique and compound were applied using rapid compound addition, holding potential was -80 mV. In general, chimera 2 currents had higher amplitudes and showed slower decay rates than chimera 1.

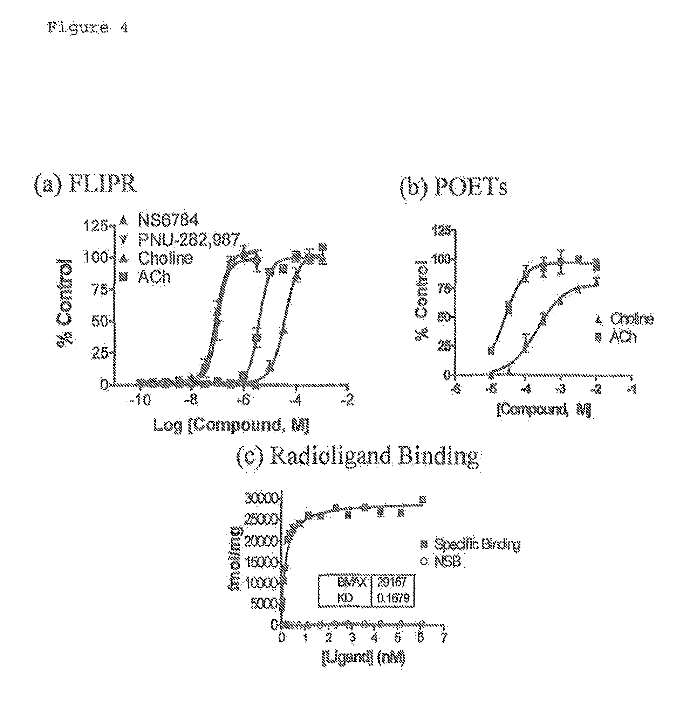

Figure 4a shows s series of concentration-responses to four agonists measured in HEK-293-chimera 2 cells using FMP dye in FLIPR. The rank order of potency is as follows: NS6784 (2-(1,4-diazabicyclo[3.2.2]nonan-4-yl)-5-phenyl-1,3,4-oxadiazole)∼ PNU-282,987 > ACh > choline. This shows that stable cell lines generated from the novel chimeras can be used to screen for agonists, antagonists, or allosteric modulators. In chimera 1 and 2 cells, the current and membrane potential responses could be evoked in concentration dependent manner by a7 agonists such as: Ach, choline, PNU-282,987, or NS6784. Figure 4b shows concentration responses to ACh and choline recorded in *Xenopus leavis* oocytes expressing chimera 2 examined using Parallel Oocyte Electrophysiology Test Station (POETs). ACh is more potent than choline similarly to what was observed in FLIPR-FMP experiments.
Figure 4c shows specific binding of [³H]-A585539 to membranes obtained from HEK-293 cells expressing chimera-1 or chimera 2. The effect of increasing unlabelled A-585539, a selective α7 agonist, on displacement of [3H]A-585539 in homogenates prepared from HEK-293-chimera 2 cells, was used for determination of affinity of this compound. As shown, [³H]A-585539 bound to a single saturable site with high affinity K_{D} equal to 0.17 nM. The Bmax was also high, 29167 fmol/mg protein, indicating high expression of chimera 2 in this cell line. Binding was high, saturable, rapid and represented >95% of total binding over the concentration range, 0.05 to 5 nM, examined. The dissociation constants (K_{D}) of 0.65 and 0.17 nM were determined for chimeras 1 and 2 respectively. The studies of electrophysiology, membrane potential measurement and radioligand binding in chimera 1 and 2 are summarized in Table 1. The comparison of potencies in chimera 1 and 2 cells illustrates that EC₅₀ values in the former were shifted to the left by 2-5-fold consistent with the observed shift in the affinity to [³H]A-585539 (Table 1). These results indicate that the chimeras, especially chimera 1 and chimera 2, function as α7 nAChR-selective ion channels and will be useful for screening various types of α7-nAChR-selective ligands including, agonists, antagonists, and allosteric modulators.

### Example 2. RESPONSES OF CHIMERAS TO POSITIVE ALLOSTERIC MODULATORS (PAMs).

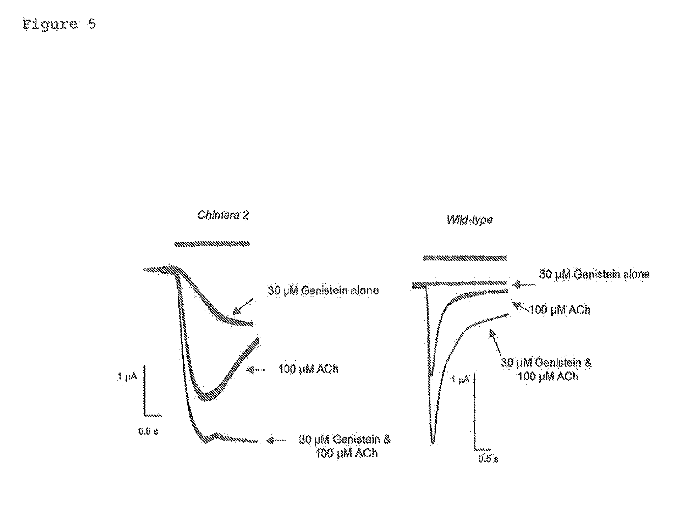

As described previously (Gronlien et al. Mol Pharmacol. 2007),genistein and 5-hydroxyindole (5-HI) potentiate α7 nAChR agonist -evoked currents by primarily increasing the current amplitude and with relatively little effect on time course of current response. These positive allosteric modulators (PAMs) were examined to determine whether these compounds could modulate the chimeras. Figures 5 - 7, show that genistein and 5-HI had differential effects on chimeras.

In chimera 2, 30 µM genistein not only potentiated peak amplitude of ACh current responses, but affected the time course of the response resulting in weakly or non-decaying decaying current. In addition, the time course of the response in chimera 2 was affected differently by genistein in comparison to the wild type α7. At the wild type α7, genistein potentiates the α7 agonist evoked a7 currents by primarily increasing the current amplitude (Figure 5).

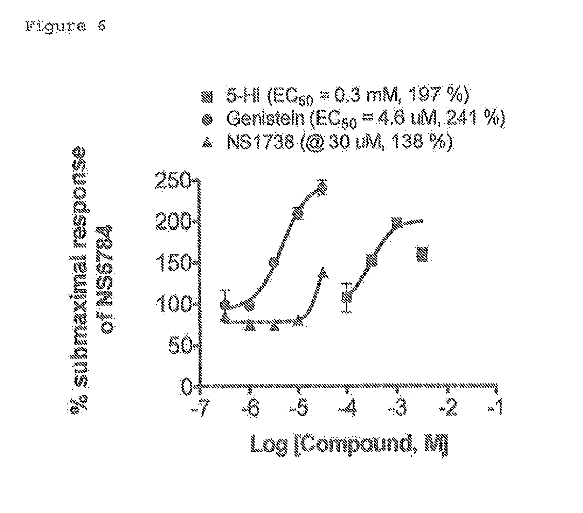

Figure 6 demonstrate that chimeras such as chimera-2 (illustrated) can be utilized for screening for novel PAMs. Concentration-responses to three α7 PAMs - 5-HI, NS1738, and genistein, potentiating submaximum NS6784 evoked responses (60 nM) in HEK-293-chimera 2 cells. The protocol employed here to determine the PAM activity is known to one skilled in the art, and involves using a submaximum concentration of a chosen α7 agonist - corresponding to EC₂₀ to EC₅₀ - such as 60 nM of NS6784 in FLIPR experiments or 100 µM ACh in Xenopus oocyte studies, and determination of concentration-dependency of test compounds to affect these submaxmium agonist signals. As shown in figure 6, reference PAMs with various potencies such as genistein, 5HI and NS1738 were identified by examining membrane potential responses in chimera-2.

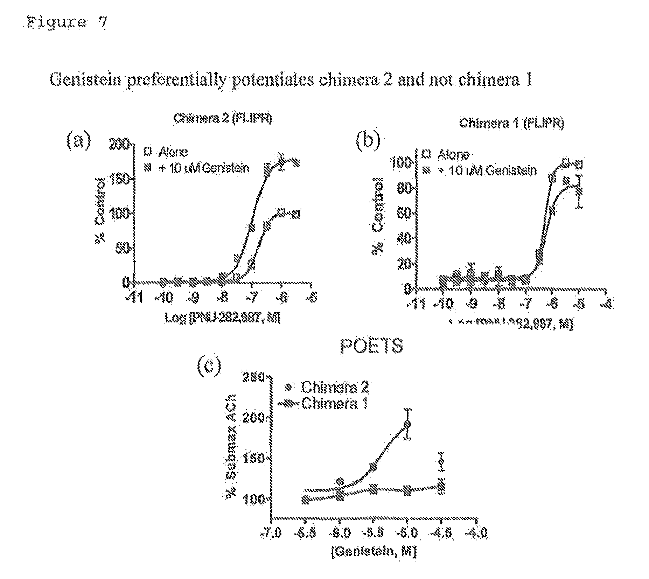

Figure 7 shows differential potentiation by genestein in chimeras 1 and 2. In chimera 1 - lacking the α7 encoded sequence for extracellular TMII-III loop - genistein was not effective as positive allosteric modulator. In contrast, in chimera 2 - containing the α7 encoded sequence for extracellular TMII-III loop - genestein was very effective. This differential potentiation of chimera 2, and not chimera 1, was confirmed electxophysiologically (see Figure 7C), wherein genestein potentiated ACh responses in chimera 2, but not chimera 1. This demonstrates that the α7-encoded sequence for extracellular TMII-III loop was critical for the positive allosteric modulation by genestein. In contrast to genestein, two other PAMs, NS1738 (Timmermann et al. J. Pharmacol. Exp. Ther. 2007) and 5-hydroxyindole, were able to potentiate both chimeras.

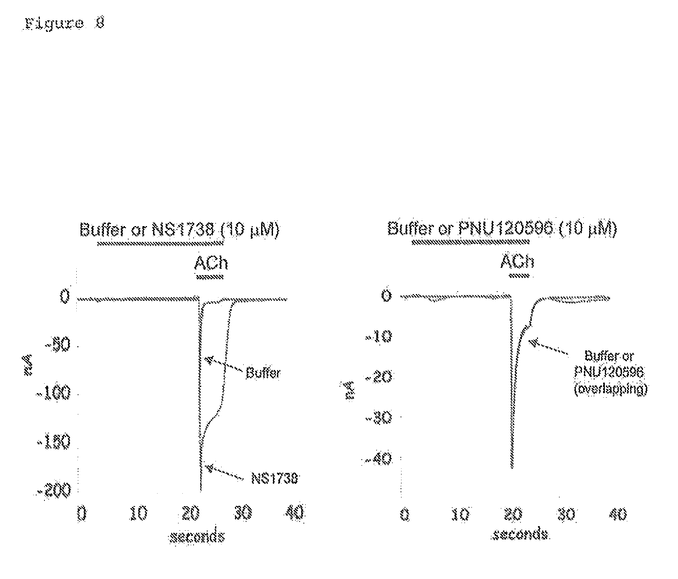

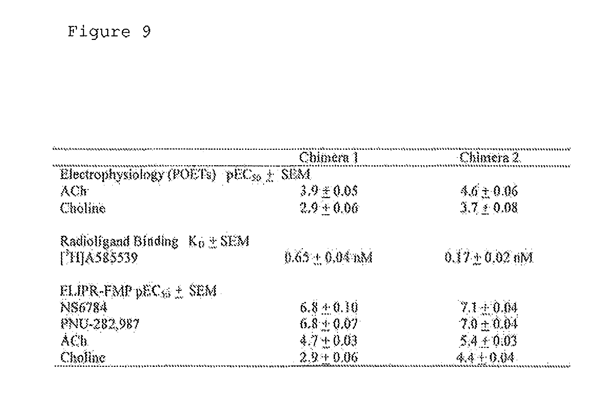

Figure 8 shows differential effects of NS1738 and PNU-120596 in chimera 1 and 2; NS1738 potentiates chimera 1, whereas PNU-120596 does not. The observation that genistein differentially potentiates chimeras offers unique opportunities to screen compounds capable of potentiating wild-type α7. Compounds such as genistein that selectively potentiate chimera (e.g. chimera 2) containing the α7 encoding TMII-III loop (e.g. chimera 2) can be identified by using this type of chimeric receptors and not when TMII-III loop is encoded by 5-HT3A. Therefore, the advantage of using these chimeras is that PAMs of certain types or pharmacological properties can be readily identified.

The invention regards the following items:
1. A recombinant nucleic acid encoding a fully human amino acid sequence comprising a cholinergic/serotoninergic chimeric receptor.
2. The recombinant nucleic acid of item 1, wherein the encoded amino acid extracellular domain has the sequence of a human neuronal nicotinic cholinergic subunit receptor, and the encoded amino acid intracellular domain has the sequence of a human serotonin receptor.
3. The recombinant nucleic acid of item further encoding for a four-transmembrane domain with an amino acid sequence of a human serotonin receptor.
4. The cholinergic/serotoninergic chimeric receptor of item 1 wherein the human neuronal nicotinic cholinergic subunit is an α7 subunit.
5. The cholinergic/serotoninergic chimeric receptor of item 1, wherein the human serotonin receptor is a 5HT3 receptor.
6. The amino acid sequence of the encoded cholinergic/serotoninergic chimeric receptor of item 1 wherein part of the sequence of the transmembrane domain is from a human neuronal nicotinic cholinergic subunit receptor.
7. The amino acid sequence of the encoded cholinergic/serotoninergic chimeric receptor of item 1 wherein the N-terminal extracellular domain is from human serotonin receptor is a 5HT3 receptor, and the transmembrane domain is from a human neuronal nicotinic cholinergic subunit receptor.
8. The nucleic acid sequence of item 1, wherein said sequence is selected from the group consisting of SEQ.ID.NO:1, SEQ.ID.NO:2, SEQ.ID.NO:3, SEQ.ID.NO:4, SEQ.ID.NO:5, SEQ.ID.NO:6, SEQ.ID.NO:7, and SEQ.ID.NO:8.
9. The amino acid sequence encoded by nucleic acid sequence of item 1, selected from the group consisting of SEQ.ID.NO:9, SEQ. ID.NO: 10, SEQ. ID.NO: 11 , SEQ.IDNO:12, SEQ.ID.NO:13, SEQ. ID.NO: 14 , SEQ. ID.NO: 15, and SEQ.ID.NO:16.
10. A vector containing the recombinant nucleic acid sequence of item 8.
11. The vector of item 10 operable linked to control sequences recognized by a host cell transformed with the vector.
12. A host cell comprising the vector of item 10.
13. The host cell of item 12 wherein said cell is a cell line derived from mammalian cells, primary mammalian cell cultures, or oocytes.
14. A fully human cholinergic/serotoninergic chimeric receptor encoded by recombinant nucleic acid sequence of item 8.
15. A method of manufacturing a chimeric receptor comprising a cholinergic/serotoninergic chimeric receptor comprising one or more regions of a human neuronal nicotinic receptor subunit and a human serotonin receptor with a vector of item 8.
16. A composition comprising a cholinergic/serotoninergic chimeric receptor comprising one or more regions of a human neuronal nicotinic receptor subunit and a human serotonin receptor.
17. The composition of item 16, wherein the chimeric receptor comprises the amino acid sequence of item 9.
18. A method of screening for compounds that bind to a region of the chimeric receptor of item 14 selected from the N-terminal domain, C-terminal domain and the extracellular loop between TM2-TM3, to modulate the activity of a neuronal nicotinic receptor.
19. The method of item 18, wherein the screening is assessed by binding or activity-based assays and determining whether the test compound binds or modulates the chimeric receptor, wherein the binding or modulation is indicative that the test compound binds or modulates the neuronal nicotinic receptor.
20. A method of screening for a compound that binds or modulates the activity of a neuronal nicotinic receptor, comprising
   - introducing a host cell expressing the chimeric receptor as specified in item 14 into an acceptable medium, and
   - monitoring an effecting said host cell indicative of binding or modulation of the test compound with the chimeric receptor, wherein the binding or modulation is indicative that the test compound binds or modulates the neuronal nicotinic receptor.
21. A kit comprising a host cell transformed or transfected with an expression vector comprising a nucleic acid sequence encoding a chimeric receptor as specified in item 14.

SEQIDNO:1 (chimera 1)
ATGCGCTGCTCGCCGGGAGGCGTCTGGCTGGCTCTGGCCGCGTCGCTGCTGCACGTGTCCCTGCAAGGCGAGTTCCA
GAGGAAGCTTTACAAGGAGCTGGTCAAGAACTACAATCCCTTGGAGAGGCCCGTGGCCAATGACTCGCAACCACTCA
CCGTCTACTTCTCCCTGAGCCTCCTGCAGATCATGGACGTGGATGAGAAGAACCAAGTTTTAACCACCAACATTTGG
CTGCAAATGTCTTGGACAGATCACTATTTACAGTGGAATGTGTCAGAATATCCAGGGGTGAAGACTGTTCGTTTCCC
AGATGGCCAGATTTGGAAACCAGACATTCTTCTCTATAACAGTGCTGATGAGCGCTTTGACGCCACATTCCACACTA
ACGTGTTGGTGAATTCTTCTGGGCATTGCCAGTACCTGCCTCCAGGCATATTCAAGAGTTCCTGCTACATCGATGTA
CGCTGGTTTCCCTTTGATGTGCAGCACTGCAAACTGAAGTTTGGGTCCTGGTCTTACGGAGGCTGGTCCTTGGATCT
GCAGATGCAGGAGGCAGATATCAGTGGCTATATCCCCAATGGAGAATGGGACCTAGTGGGAATCCCCGGCAAGAGGA
GTGAAAGGTTCTATGAGTGCTGCAAAGAGCCCTACCCCGATGTCACCTTCACAGTGGTCATCCGACGTAGGCCACTC
TTCTATGTGGTCAGCCTGCTACTGCCCAGCATCTTCCTCATGGTCATGGACATCGTGGGCTTCTACCTGCCCCCCAA
CAGTGGCGAGAGGGTCTCTTTCAAGATTACACTCCTCCTGGGCTACTCGGTCTTCCTGATCATCGTTTCTGACACGC
TGCCGGCCACTGCCATCGGCACTCCTCTCATTGGTGTCTACTTTGTGGTGTGCATGGCTCTGCTGGTGATAAGTTTG
GCCGAGACCATCTTCATTGTGCGGCTGGTGCACAAGCAAGACCTGCAGCAGCCCGTGCCTGCTTGGCTGCGTCACCT
GGTTCTGGAGAGAATCGCCTGGCTACTTTGCCTGAGGGAGCAGTCAACTTCCCAGAGGCCCCCAGCCACCTCCCAAG
CCACCAAGACTGATGACTGCTCAGCCATGGGAAACCACTGCAGCCACATGGGAGGACCCCAGGACTTCGAGAAGAGC
CCGAGGGACAGATGTAGCCCTCCCCCACCACCTCGGGAGGCCTCGCTGGCGGTGTGTGGGCTGCTGCAGGAGCTGTC
CTCCATCCGGCAATTCCTGGAAAAGCGGGATGAGATCCGAGAGGTGGCCCGAGACTGGCTGCGCGTGGGCTCCGTGC
TGGACAAGCTGCTATTCCACATTTACCTGCTAGCGGTGCTGGCCTACAGCATCACCCTGGTTATGCTCTGGTCCATC
TGGCAGTACGCTTGA

SEQIDNO:2 (chimera 2)
ATGCGCTGCTCGCCGGGAGGCGTCTGGCTGGCTCTGGCCGCGTCGCTGCTGCACGTGTCCCTGCAAGGCGAGTTCCA
GAGGAAGCTTTACAAGGAGCTGGTCAAGAACTACAATCCCTTGGAGAGGCCCGTGGCCAATGACTCGCAACCACTCA
CCGTCTACTTCTCCCTGAGCCTCCTGCAGATCATGGACGTGGATGAGAAGAACCAAGTTTTAACCACCAACATTTGG
CTGCAAATGTCTTGGACAGATCACTATTTACAGTGGAATGTGTCAGAATATCCAGGGGTGAAGACTGTTCGTTTCCC
AGATGGCCAGATTTGGAAACCAGACATTCTTCTCTATAACAGTGCTGATGAGCGCTTTGACGCCACATTCCACACTA
ACGTGTTGGTGAATTCTTCTGGGCATTGCCAGTACCTGCCTCCAGGCATATTCAAGAGTTCCTGCTACATCGATGTA
CGCTGGTTTCCCTTTGATGTGCAGCACTGCAAACTGAAGTTTGGGTCCTGGTCTTACGGAGGCTGGTCCTTGGATCT
GCAGATGCAGGAGGCAGATATCAGTGGCTATATCCCCAATGGAGAATGGGACCTAGTGGGAATCCCCGGCAAGAGGA
GTGAAAGGTTCTATGAGTGCTGCAAAGAGCCCTACCCCGATGTCACCTTCACAGTGGTCATCCGACGTAGGCCACTC
TTCTATGTGGTCAGCCTGCTACTGCCCAGCATCTTCCTCATGGTCATGGACATCGTGGGCTTCTACCTGCCCCCCAA
CAGTGGCGAGAGGGTCTCTTTCAAGATTACACTCCTCCTGGGCTACTCGGTCTTCCTGATCATCGTTGCTGAGATCA
TGCCCGCAACATCCGATTCGACTCCTCTCATTGGTGTCTACTTTGTGGTGTGCATGGCTCTGCTGGTGATAAGTTTG
GCCGAGACCATCTTCATTGTGCGGCTGGTGCACAAGCAAGACCTGCAGCAGCCCGTGCCTGCTTGGCTGCGTCACCT
GGTTCTGGAGAGAATCGCCTGGCTACTTTGCCTGAGGGAGCAGTCAACTTCCCAGAGGCCCCCAGCCACCTCCCAAG
CCACCAAGACTGATGACTGCTCAGCCATGGGAAACCACTGCAGCCACATGGGAGGACCCCAGGACTTCGAGAAGAGC
CCGAGGGACAGATGTAGCCCTCCCCCACCACCTCGGGAGGCCTCGCTGGCGGTGTGTGGGCTGCTGCAGGAGCTGTC
CTCCATCCGGCAATTCCTGGAAAAGCGGGATGAGATCCGAGAGGTGGCCCGAGACTGGCTGCGCGTGGGCTCCGTGC
TGGACAAGCTGCTATTCCACATTTACCTGCTAGCGGTGCTGGCCTACAGCATCACCCTGGTTATGCTCTGGTCCATC
TGGCAGTACGCTTGA

SEQIDNO:3 (chimera 3)
ATGCGCTGCTCGCCGGGAGGCGTCTGGCTGGCTCTGGCCGCGTCGCTGCTGCACGTGTCCCTGCAAGGCGAGTTCCA
GAGGAAGCTTTACAAGGAGCTGGTCAAGAACTACAATCCCTTGGAGAGGCCCGTGGCCAATGACTCGCAACCACTCA
CCGTCTACTTCTCCCTGAGCCTCCTGCAGATCATGGACGTGGATGAGAAGAACCAAGTTTTAACCACCAACATTTGG
CTGCAAATGTCTTGGACAGATCACTATTTACAGTGGAATGTGTCAGAATATCCAGGGGTGAAGACTGTTCGTTTCCC
AGATGGCCAGATTTGGAAACCAGACATTCTTCTCTATAACAGTGCTGATGAGCGCTTTGACGCCACATTCCACACTA
ACGTGTTGGTGAATTCTTCTGGGCATTGCCAGTACCTGCCTCCAGGCATATTCAAGAGTTCCTGCTACATCGATGTA
CGCTGGTTTCCCTTTGATGTGCAGCACTGCAAACTGAAGTTTGGGTCCTGGTCTTACGGAGGCTGGTCCTTGGATCT
GCAGATGCAGGAGGCAGATATCAGTGGCTATATCCCCAATGGAGAATGGGACCTAGTGGGAATCCCCGGCAAGAGGA
GTGAAAGGTTCTATGAGTGCTGCAAAGAGCCCTACCCCGATGTCACCTTCACAGTGGTCATCCGACGTAGGCCACTC
TTCTATGTGGTCAGCCTGCTACTGCCCAGCATCTTCCTCATGGTCATGGACATCGTGGGCTTCTACCTGCCCCCCAA
CAGTGGCGAGAGGGTCTCTTTCAAGATTACACTCCTCCTGGGCTACTCGGTCTTCCTGATCATCGTTGCTGAGATCA
TGCCCGCAACATCCGATTCGACTCCTCTCATTGGTGTCTACTTTGTGGTGTGCATGGCTCTGCTGGTGATAAGTTTG
GCCGAGACCATCTTCATTGTGCGGCTGGTGCACAAGCAAGACCTGCAGCAGCCCGTGCCTGCTTGGCTGCGTCACCT
GGTTCTGGAGAGAATCGCCTGGCTACTTTGCCTGAGGGAGCAGTCAACTTCCCAGAGGCCCCCAGCCACCTCCCAAG
CCACCAAGACTGATGACTGCTCAGCCATGGGAAACCACTGCAGCCACATGGGAGGACCCCAGGACTTCGAGAAGAGC
CCGAGGGACAGATGTAGCCCTCCCCCACCACCTCGGGAGGCCTCGCTGGCGGTGTGTGGGCTGCTGCAGGAGCTGTC
CTCCATCCGGCAATTCCTGGAAAAGCGGGATGAGATCCGAGAGGTGGCCCGAGACTGGCTGCGCGTGGGCTCCGTGC
TGGACAAGCTGCTATTCCACATTTACCTGCTAGCGGTGCTGGCCTACAGCATCACCCTGGTTATGCTCTGGTCCATC
TGGGTGGAGGCCGTGTCCAAAGACTTTGCGTGA

SEQIDNO:4 (chimera 4)
ATGCGCTGCTCGCCGGGAGGCGTCTGGCTGGCTCTGGCCGCGTCGCTGCTGCACGTGTCCCTGCAAGGCGAGTTCCA
GAGGAAGCTTTACAAGGAGCTGGTCAAGAACTACAATCCCTTGGAGAGGCCCGTGGCCAATGACTCGCAACCACTCA
CCGTCTACTTCTCCCTGAGCCTCCTGCAGATCATGGACGTGGATGAGAAGAACCAAGTTTTAACCACCAACATTTGG
CTGCAAATGTCTTGGACAGATCACTATTTACAGTGGAATGTGTCAGAATATCCAGGGGTGAAGACTGTTCGTTTCCC
AGATGGCCAGATTTGGAAACCAGACATTCTTCTCTATAACAGTGCTGATGAGCGCTTTGACGCCACATTCCACACTA
ACGTGTTGGTGAATTCTTCTGGGCATTGCCAGTACCTGCCTCCAGGCATATTCAAGAGTTCCTGCTACATCGATGTA
CGCTGGTTTCCCTTTGATGTGCAGCACTGCAAACTGAAGTTTGGGTCCTGGTCTTACGGAGGCTGGTCCTTGGATCT
GCAGATGCAGGAGGCAGATATCAGTGGCTATATCCCCAATGGAGAATGGGACCTAGTGGGAATCCCCGGCAAGAGGA
GTGAAAGGTTCTATGAGTGCTGCAAAGAGCCCTACCCCGATGTCACCTTCACAGTGGTCATCCGACGTAGGCCACTC
TTCTATGTGGTCAGCCTGCTACTGCCCAGCATCTTCCTCATGGTCATGGACATCGTGGGCTTCTACCTGCCCCCCAA
CAGTGGCGAGAGGGTCTCTTTCAAGATTACACTCCTCCTGGGCTACTCGGTCTTCCTGATCATCGTTTCTGACACGC
TGCCGGCCACTGCCATCGGCACTCCTCTCATTGGTGTCTACTTTGTGGTGTGCATGGCTCTGCTGGTGATAAGTTTG
GCCGAGACAGTGATCGTGCTGCAGTACCACCACCACGACCCCGACGGGGGCAAGATGCCCAAGTGGACCAGAGTCAT
CCTTCTGAACTGGTGCGCGTGGTTCCTGCGAATGAAGAGGCCCGGGGAGGACAAGGTGCGCCCGGCCTGCCAGCACA
AGCAGCGGCGCTGCAGCCTGGCCAGTGTGGAGATGAGCGCCGTGGCCCCGCCGCCCGCCAGCAACGGGAACCTGCTG
TACATCGGCTTCCGCGGCCTGGACGGCGTGCACTGTGTCCCGACCCCCGACTCTGGGGTAGTGTGTGGCCGCATGGC
CTGCTCCCCCACGCACGATGAGCACCTCCTGCACGGCGGGCAACCCCCCGAGGGGGACCCGGACTTGGCCAAGATCC
TGGAGGAGGTCCGCTACATTGCCAACCGCTTCCGCTGCCAGGACGAAAGCGAGGCGGTCTGCAGCGAGTGGAAGTTC
GCCGCCTGTGTGGTGGACAAGCTGCTATTCCACATTTACCTGCTAGCGGTGCTGGCCTACAGCATCACCCTGGTTAT
GCTCTGGTCCATCTGGCAGTACGCTTGA

SEQIDNO:5 (chimera 5)
ATGCGCTGCTCGCCGGGAGGCGTCTGGCTGGCTCTGGCCGCGTCGCTGCTGCACGTGTCCCTGCAAGGCGAGTTCCA
GAGGAAGCTTTACAAGGAGCTGGTCAAGAACTACAATCCCTTGGAGAGGCCCGTGGCCAATGACTCGCAACCACTCA
CCGTCTACTTCTCCCTGAGCCTCCTGCAGATCATGGACGTGGATGAGAAGAACCAAGTTTTAACCACCAACATTTGG
CTGCAAATGTCTTGGACAGATCACTATTTACAGTGGAATGTGTCAGAATATCCAGGGGTGAAGACTGTTCGTTTCCC
AGATGGCCAGATTTGGAAACCAGACATTCTTCTCTATAACAGTGCTGATGAGCGCTTTGACGCCACATTCCACACTA
ACGTGTTGGTGAATTCTTCTGGGCATTGCCAGTACCTGCCTCCAGGCATATTCAAGAGTTCCTGCTACATCGATGTA
CGCTGGTTTCCCTTTGATGTGCAGCACTGCAAACTGAAGTTTGGGTCCTGGTCTTACGGAGGCTGGTCCTTGGATCT
GCAGATGCAGGAGGCAGATATCAGTGGCTATATCCCCAATGGAGAATGGGACCTAGTGGGAATCCCCGGCAAGAGGA
GTGAAAGGTTCTATGAGTGCTGCAAAGAGCCCTACCCCGATGTCACCTTCACAGTGGTCATCCGACGTAGGCCACTC
TTCTATGTGGTCAGCCTGCTACTGCCCAGCATCTTCCTCATGGTCATGGACATCGTGGGCTTCTACCTGCCCCCCAA
CAGTGGCGAGAGGGTCTCTTTCAAGATTACACTCCTCCTGGGCTACTCGGTCTTCCTGATCATCGTTGCTGAGATCA
TGCCCGCAACATCCGATTCGACTCCTCTCATTGGTGTCTACTTTGTGGTGTGCATGGCTCTGCTGGTGATAAGTTTG
GCCGAGACAGTGATCGTGCTGCAGTACCACCACCACGACCCCGACGGGGGCAAGATGCCCAAGTGGACCAGAGTCAT
CCTTCTGAACTGGTGCGCGTGGTTCCTGCGAATGAAGAGGCCCGGGGAGGACAAGGTGCGCCCGGCCTGCCAGCACA
AGCAGCGGCGCTGCAGCCTGGCCAGTGTGGAGATGAGCGCCGTGGCCCCGCCGCCCGCCAGCAACGGGAACCTGCTG
TACATCGGCTTCCGCGGCCTGGACGGCGTGCACTGTGTCCCGACCCCCGACTCTGGGGTAGTGTGTGGCCGCATGGC
CTGCTCCCCCACGCACGATGAGCACCTCCTGCACGGCGGGCAACCCCCCGAGGGGGACCCGGACTTGGCCAAGATCC
TGGAGGAGGTCCGCTACATTGCCAACCGCTTCCGCTGCCAGGACGAAAGCGAGGCGGTCTGCAGCGAGTGGAAGTTC
GCCGCCTGTGTGGTGGACAAGCTGCTATTCCACATTTACCTGCTAGCGGTGCTGGCCTACAGCATCACCCTGGTTAT
GCTCTGGTCCATCTGGCAGTACGCTTGA

SEQIDNO:6 (chimera 6)
ATGCGCTGCTCGCCGGGAGGCGTCTGGCTGGCTCTGGCCGCGTCGCTGCTGCACGTGTCCCTGCAAGGCGAGTTCCA
GAGGAAGCTTTACAAGGAGCTGGTCAAGAACTACAATCCCTTGGAGAGGCCCGTGGCCAATGACTCGCAACCACTCA
CCGTCTACTTCTCCCTGAGCCTCCTGCAGATCATGGACGTGGATGAGAAGAACCAAGTTTTAACCACCAACATTTGG
CTGCAAATGTCTTGGACAGATCACTATTTACAGTGGAATGTGTCAGAATATCCAGGGGTGAAGACTGTTCGTTTCCC
AGATGGCCAGATTTGGAAACCAGACATTCTTCTCTATAACAGTGCTGATGAGCGCTTTGACGCCACATTCCACACTA
ACGTGTTGGTGAATTCTTCTGGGCATTGCCAGTACCTGCCTCCAGGCATATTCAAGAGTTCCTGCTACATCGATGTA
CGCTGGTTTCCCTTTGATGTGCAGCACTGCAAACTGAAGTTTGGGTCCTGGTCTTACGGAGGCTGGTCCTTGGATCT
GCAGATGCAGGAGGCAGATATCAGTGGCTATATCCCCAATGGAGAATGGGACCTAGTGGGAATCCCCGGCAAGAGGA
GTGAAAGGTTCTATGAGTGCTGCAAAGAGCCCTACCCCGATGTCACCTTCACAGTGGTCATCCGACGTAGGCCACTC
TTCTATGTGGTCAGCCTGCTACTGCCCAGCATCTTCCTCATGGTCATGGACATCGTGGGCTTCTACCTGCCCCCCAA
CAGTGGCGAGAGGGTCTCTTTCAAGATTACACTCCTCCTGGGCTACTCGGTCTTCCTGATCATCGTTGCTGAGATCA
TGCCCGCAACATCCGATTCGACTCCTCTCATTGGTGTCTACTTTGTGGTGTGCATGGCTCTGCTGGTGATAAGTTTG
GCCGAGACAGTGATCGTGCTGCAGTACCACCACCACGACCCCGACGGGGGCAAGATGCCCAAGTGGACCAGAGTCAT
CCTTCTGAACTGGTGCGCGTGGTTCCTGCGAATGAAGAGGCCCGGGGAGGACAAGGTGCGCCCGGCCTGCCAGCACA
AGCAGCGGCGCTGCAGCCTGGCCAGTGTGGAGATGAGCGCCGTGGCCCCGCCGCCCGCCAGCAACGGGAACCTGCTG
TACATCGGCTTCCGCGGCCTGGACGGCGTGCACTGTGTCCCGACCCCCGACTCTGGGGTAGTGTGTGGCCGCATGGC
CTGCTCCCCCACGCACGATGAGCACCTCCTGCACGGCGGGCAACCCCCCGAGGGGGACCCGGACTTGGCCAAGATCC
TGGAGGAGGTCCGCTACATTGCCAACCGCTTCCGCTGCCAGGACGAAAGCGAGGCGGTCTGCAGCGAGTGGAAGTTC

GCCGCCTGTGTGGTGGACAAGCTGCTATTCCACATTTACCTGCTAGCGGTGCTGGCCTACAGCATCACCCTGGTTAT
GCTCTGGTCCATCTGGGTGGAGGCCGTGTCCAAAGACTTTGCGTGA

SEQIDNO:7 (chimera 7)
ATGCGCTGCTCGCCGGGAGGCGTCTGGCTGGCTCTGGCCGCGTCGCTGCTGCACGTGTCCCTGCAAGGCGAGTTCCA
GAGGAAGCTTTACAAGGAGCTGGTCAAGAACTACAATCCCTTGGAGAGGCCCGTGGCCAATGACTCGCAACCACTCA
CCGTCTACTTCTCCCTGAGCCTCCTGCAGATCATGGACGTGGATGAGAAGAACCAAGTTTTAACCACCAACATTTGG
CTGCAAATGTCTTGGACAGATCACTATTTACAGTGGAATGTGTCAGAATATCCAGGGGTGAAGACTGTTCGTTTCCC
AGATGGCCAGATTTGGAAACCAGACATTCTTCTCTATAACAGTGCTGATGAGCGCTTTGACGCCACATTCCACACTA
ACGTGTTGGTGAATTCTTCTGGGCATTGCCAGTACCTGCCTCCAGGCATATTCAAGAGTTCCTGCTACATCGATGTA
CGCTGGTTTCCCTTTGATGTGCAGCACTGCAAACTGAAGTTTGGGTCCTGGTCTTACGGAGGCTGGTCCTTGGATCT
GCAGATGCAGGAGGCAGATATCAGTGGCTATATCCCCAATGGAGAATGGGACCTAGTGGGAATCCCCGGCAAGAGGA
GTGAAAGGTTCTATGAGTGCTGCAAAGAGCCCTACCCCGATGTCACCTTCACAGTGGTCATCCGACGTAGGCCACTC
TTCTATGTGGTCAGCCTGCTACTGCCCAGCATCTTCCTCATGGTCATGGACATCGTGGGCTTCTACCTGCCCCCCAA
CAGTGGCGAGAGGGTCTCTTTCAAGATTACACTCCTCCTGGGCTACTCGGTCTTCCTGATCATCGTTTCTGACACGC
TGCCGGCCACTGCCATCGGCACTCCTCTCATTGGTGTCTACTTTGTGGTGTGCATGGCTCTGCTGGTGATAAGTTTG
GCCGAGACAGTGATCGTGCTGCAGTACCACCACCACGACCCCGACGGGGGCAAGATGCCCAAGTGGACCAGAGTCAT
CCTTCTGAACTGGTGCGCGTGGTTCCTGCGAATGAAGAGGCCCGGGGAGGACAAGGTGCGCCCGGCCTGCCAGCACA
AGCAGCGGCGCTGCAGCCTGGCCAGTGTGGAGATGAGCGCCGTGGGCCCGCCGCCCGCCAGCAACGGGAACCTGCTG
TACATCGGCTTCCGCGGCCTGGACGGCGTGCACTGTGTCCCGACCCCCGACTCTGGGGTAGTGTGTGGCCGCATGGC
CTGCTCCCCCACGCACGATGAGCACCTCCTGCACGGCGGGCAACCCCCCGAGGGGGACCCGGACTTGGCCAAGATCC
TGGAGGAGGTCCGCTACATTGCCAACCGCTTCCGCTGCCAGGACGAAAGCGAGGCGGTCTGCAGCGAGTGGAAGTTC
GCCGCCTGTGTGGTGGACAAGCTGCTATTCCACATTTACCTGCTAGCGGTGCTGGCCTACAGCATCACCCTGGTTAT
GCTCTGGTCCATCTGGGTGGAGGCCGTGTCCAAAGACTTTGCGTGA

SEQIDNO:8 (chimera 8)
ATGCTTGGAAAGCTCGCTATGCTGCTGTGGGTCCAGCAGGCGCTGCTCGCCTTGCTCCTCCCCACACTCCTGGCACA
GGGAGAAGCCAGGAGGAGCCGAAACACCACCAGGCCCGCTCTGCTGAGGCTGTCGGATTACCTTTTGACCAACTACA
GGAAGGGTGTGCGCCCCGTGAGGGACTGGAGGAAGCCAACCACCGTATCCATTGACGTCATTGTCTATGCCATCCTC
AACGTGGATGAGAAGAATCAGGTGCTGACCACCTACATCTGGTACCGGCAGTACTGGACTGATGAGTTTCTCCAGTG
GAACCCTGAGGACTTTGACAACATCACCAAGTTGTCCATCCCCACGGACAGCATCTGGGTCCCGGACATTCTCATCA
ATGAGTTCGTGGATGTGGGGAAGTCTCCAAATATCCCGTACGTGTATATTCGGCATCAAGGCGAAGTTCAGAACTAC
AAGCCCCTTCAGGTGGTGACTGCCTGTAGCCTCGACATCTACAACTTCCCCTTCGATGTCCAGAACTGCTCGCTGAC
CTTCACCAGTTGGCTGCACACCATCCAGGACATCAACATCTCTTTGTGGCGCTTGCCAGAAAAGGTGAAATCCGACA
GGAGTGTCTTCATGAACCAGGGAGAGTGGGAGTTGCTGGGGGTGCTGCCCTACTTTCGGGAGTTCAGCATGGAAAGC
AGTAACTACTATGCAGAAATGAAGTTCTATGTGACCATGCGCCGCAGGACGCTCTACTATGGCCTCAACCTGCTGAT
CCCCTGTGTGCTCATCTCCGCCCTCGCCCTGCTGGTGTTCCTGCTTCCTGCAGATTCCGGGGAGAAGATTTCCCTGG
GGATAACAGTCTTACTCTCTCTTACCGTCTTCATGCTGCTCGTGGCTGAGATCATGCCCGCAACATCCGATTCGGTA
CCATTGATAGCCCAGTACTTCGCCAGCACCATGATCATCGTGGGCCTCTCGGTGGTGGTGACGGTGATCGTGCTGCA
GTACCACCACCACGACCCCGACGGGGGCAAGATGCCCAAGTGGACCAGAGTCATCCTTCTGAACTGGTGCGCGTGGT
TCCTGCGAATGAAGAGGCCCGGGGAGGACAAGGTGCGCCCGGCCTGCCAGCACAAGCAGCGGCGCTGCAGCCTGGCC
AGTGTGGAGATGAGCGCCGTGGCGCCGCCGCCCGCCAGCAACGGGAACCTGCTGTACATCGGCTTCCGCGGCCTGGA
CGGCGTGCACTGTGTCCCGACCCCCGACTCTGGGGTAGTGTGTGGCCGCATGGCCTGCTCCCCCACGCACGATGAGC
ACCTCCTGCACGGCGGGCAACCCCCCGAGGGGGACCCGGACTTGGCCAAGATCCTGGAGGAGGTCCGCTACATTGCC
AACCGCTTCCGCTGCCAGGACGAAAGCGAGGCGGTCTGCAGCGAGTGGAAGTTCGCCGCCTGTGTGGTGGACCGCCT
GTGCCTCATGGCCTTCTCGGTCTTCACCATCATCTGCACCATCGGCATCCTGATGTCGGCTCCCAACTTCGTGGAGG
CCGTGTCCAAAGACTTTGCGTAA

SEQIDNO:9 (chimera 1)
MRCSPGGVWLALAASLLHVSLQGEFQRKLYKELVKNYNPLERPVANDSQPLTVYFSLSLLQIMDVDEKNQVLTTNIW
LQMSWTDHYLQWNVSEYPGVKTVRFPDGQIWKPDILLYNSADERFDATFHTNVLVNSSGHCQYLPPGIFKSSCYIDV
RWFPFDVQHCKLKFGSWSYGGWSLDLQMQEADISGYIPNGEWDLVGIPGKRSERFYECCKEPYPDVTFTVVIRRRPL
FYVVSLLLPSIFLMVMDIVGFYLPPNSGERVSFKITLLLGYSVFLIIVSDTLPATAIGTPLIGVYFVVCMALLVISL
AETIFIVRLVHKQDLQQPVPAWLRHLVLERIAWLLCLREQSTSQRPPATSQATKTDDCSAMGNHCSHMGGPQDFEKS
PRDRCSPPPPPREASLAVCGLLQELSSIRQFLEKRDEIREVARDWLRVGSVLDKLLFHIYLLAVLAYSITLVMLWSI
WQYA

SEQIDNO:10 (chimera 2)
MRCSPGGVWLALAASLLHVSLQGEFQRKLYKELVKNYNPLERPVANDSQPLTVYFSLSLLQIMDVDEKNQVLTTNIW
LQMSWTDHYLQWNVSEYPGVKTVRFPDGQIWKPDILLYNSADERFDATFHTNVLVNSSGHCQYLPPGIFKSSCYIDV
RWFPFDVQHCKLKFGSWSYGGWSLDLQMQEADISGYIPNGEWDLVGIPGKRSERFYECCKEPYPDVTFTVVIRRRPL
FYVVSLLLPSIFLMVMDIVGFYLPPNSGERVSFKITLLLGYSVFLIIVAEIMPATSDSTPLIGVYFVVCMALLVISL
AETIFIVRLVHKQDLQQPVPAWLRHLVLERIAWLLCLREQSTSQRPPATSQATKTDDCSAMGNHCSHMGGPQDFEKS

PRDRCSPPPPPREASLAVCGLLQELSSIRQFLEKRDEIREVARDWLRVGSVLDKLLFHIYLLAVLAYSITLVMLWSI
WQYA

SEQIDNO:11 (chimera 3)
MRCSPGGVWLALAASLLHVSLQGEFQRKLYKELVKNYNPLERPVANDSQPLTVYFSLSLLQIMDVDEKNQVLTTNIW
LQMSWTDHYLQWNVSEYPGVKTVRFPDGQIWKPDILLYNSADERFDATFHTNVLVNSSGHCQYLPPGIFKSSCYIDV
RWFPFDVQHCKLKFGSWSYGGWSLDLQMQEADISGYIPNGEWDLVGIPGKRSERFYECCKEPYPDVTFTVVIRRRPL
FYVVSLLLPSIFLMVMDIVGFYLPPNSGERVSFKITLLLGYSVFLIIVAEIMPATSDSTPLIGVYFVVCMALLVISL
AETIFIVRLVHKQDLQQPVPAWLRHLVLERIAWLLCLREQSTSQRPPATSQATKTDDCSAMGNHCSHMGGPQDFEKS
PRDRCSPPPPPREASLAVCGLLQELSSIRQFLEKRDEIREVARDWLRVGSVLDKLLFHIYLLAVLAYSITLVMLWSI
WVEAVSKDFA

SEQIDNO:12 (chimera 4)
MRCSPGGVWLALAASLLHVSLQGEFQRKLYKELVKNYNPLERPVANDSQPLTVYFSLSLLQIMDVDEKNQVLTTNIW
LQMSWTDHYLQWNVSEYPGVKTVRFPDGQIWKPDILLYNSADERFDATFHTNVLVNSSGHCQYLPPGIFKSSCYIDV
RWFPFDVQHCKLKFGSWSYGGWSLDLQMQEADISGYIPNGEWDLVGIPGKRSERFYECCKEPYPDVTFTVVIRRRPL
FYVVSLLLPSIFLMVMDIVGFYLPPNSGERVSFKITLLLGYSVFLIIVSDTLPATAIGTPLIGVYFVVCMALLVISL
AETVIVLQYHHHDPDGGKMPKWTRVILLNWCAWFLRMKRPGEDKVRPACQHKQRRCSLASVEMSAVAPPPASNGNLL
YIGFRGLDGVHCVPTPDSGVVCGRMACSPTHDEHLLHGGQPPEGDPDLAKILEEVRYIANRFRCQDESEAVCSEWKF
AACVVDKLLFHIYLLAVLAYSITLVMLWSIWQYA

SEQIDNO:13 (chimera 5)
MRCSPGGVWLALAASLLHVSLQGEFQRKLYKELVKNYNPLERPVANDSQPLTVYFSLSLLQIMDVDEKNQVLTTNIW
LQMSWTDHYLQWNVSEYPGVKTVRFPDGQIWKPDILLYNSADERFDATFHTNVLVNSSGHCQYLPPGIFKSSCYIDV
RWFPFDVQHCKLKFGSWSYGGWSLDLQMQEADISGYIPNGEWDLVGIPGKRSERFYECCKEPYPDVTFTVVIRRRPL
FYVVSLLLPSIFLMVMDIVGFYLPPNSGERVSFKITLLLGYSVFLIIVAEIMPATSDSTPLIGVYFVVCMALLVISL
AETVIVLQYHHHDPDGGKMPKWTRVILLNWCAWFLRMKRPGEDKVRPACQHKQRRCSLASVEMSAVAPPPASNGNLL
YIGFRGLDGVHCVPTPDSGVVCGRMACSPTHDEHLLHGGQPPEGDPDLAKILEEVRYIANRFRCQDESEAVCSEWKF
AACVVDKLLFHIYLLAVLAYSITLVMLWSIWQYA

SEQIDNO:14 (chimera 6)
MRCSPGGVWLALAASLLHVSLQGEFQRKLYKELVKNYNPLERPVANDSQPLTVYFSLSLLQIMDVDEKNQVLTTNIW
LQMSWTDHYLQWNVSEYPGVKTVRFPDGQIWKPDILLYNSADERFDATFHTNVLVNSSGHCQYLPPGIFKSSCYIDV
RWFPFDVQHCKLKFGSWSYGGWSLDLQMQEADISGYIPNGEWDLVGIPGKRSERFYECCKEPYPDVTFTVVIRRRPL
FYVVSLLLPSIFLMVMDIVGFYLPPNSGERVSFKITLLLGYSVFLIIVAEIMPATSDSTPLIGVYFVVCMALLVISL
AETVIVLQYHHHDPDGGKMPKWTRVILLNWCAWFLRMKRPGEDKVRPACQHKQRRCSLASVEMSAVAPPPASNGNLL
YIGFRGLDGVHCVPTPDSGVVCGRMACSPTHDEHLLHGGQPPEGDPDLAKILEEVRYIANRFRCQDESEAVCSEWKF
AACVVDKLLFHIYLLAVLAYSITLVMLWSIWVEAVSKDFA

SEQIDNO:15 (chimera 7)
MRCSPGGVWLALAASLLHVSLQGEFQRKLYKELVKNYNPLERPVANDSQPLTVYFSLSLLQIMDVDEKNQVLTTNIW
LQMSWTDHYLQWNVSEYPGVKTVRFPDGQIWKPDILLYNSADERFDATFHTNVLVNSSGHCQYLPPGIFKSSCYIDV
RWFPFDVQHCKLKFGSWSYGGWSLDLQMQEADISGYIPNGEWDLVGIPGKRSERFYECCKEPYPDVTFTVVIRRRPL
FYVVSLLLPSIFLMVMDIVGFYLPPNSGERVSFKITLLLGYSVFLIIVSDTLPATAIGTPLIGVYFVVCMALLVISL
AETVIVLQYHHHDPDGGKMPKWTRVILLNWCAWFLRMKRPGEDKVRPACQHKQRRCSLASVEMSAVAPPPASNGNLL
YIGFRGLDGVHCVPTPDSGVVCGRMACSPTHDEHLLHGGQPPEGDPDLAKILEEVRYIANRFRCQDESEAVCSEWKF
AACVVDKLLFHIYLLAVLAYSITLVMLWSIWVEAVSKDFA.

SEQIDNO:16 (chimera 8)
MLGKLAMLLWVQQALLALLLPTLLAQGEARRSRNTTRPALLRLSDYLLTNYRKGVRPVRDWRKPTTVSIDVIVYAIL
NVDEKNQVLTTYIWYRQYWTDEFLQWNPEDFDNITKLSIPTDSIWVPDILINEFVDVGKSPNIPYVYIRHQGEVQNY
KPLQVVTACSLDIYNFPFDVQNCSLTFTSWLHTIQDINISLWRLPEKVKSDRSVFMNQGEWELLGVLPYFREFSMES
SNYYAEMKFYVTMRRRTLYYGLNLLIPCVLISALALLVFLLPADSGEKISLGITVLLSLTVFMLLVAEIMPATSDSV
PLIAQYFASTMIIVGLSVVVTVIVLQYHHHDPDGGKMPKWTRVILLNWCAWFLRMKRPGEDKVRPACQHKQRRCSLA
SVEMSAVAPPPASNGNLLYIGFRGLDGVHCVPTPDSGVVCGRMACSPTHDEHLLHGGQPPEGDPDLAKILEEVRYIA
NRFRCQDESEAVCSEWKFAACVVDRLCLMAFSVFTIICTIGILMSAPNFVEAVSKDFA

## Claims

1. A recombinant nucleic acid encoding a cholinergic/serotoninergic chimeric receptor, wherein the recombinant nucleic acid comprises SEQ ID NO: 2.

2. The recombinant nucleic acid of claim 1, wherein the recombinant nucleic acid encodes a polypeptide having an amino acid sequence of SEQ ID NO: 10.

3. A vector comprising the recombinant nucleic acid of claim 1.

4. The vector of claim 3, wherein the recombinant nucleic acid is operably linked to control sequences recognized by a host cell transformed with the vector.

5. A host cell comprising the vector of claim 3.

6. The host cell of claim 5, wherein the host cell is a cell line derived from mammalian cells, primary mammalian cell cultures, or oocytes.

7. A cholinergic/serotoninergic chimeric receptor encoded by the recombinant nucleic acid sequence of claim 1.

8. A composition comprising a cholinergic/serotoninergic chimeric receptor comprising the amino acid sequence of SEQ ID NO: 10.

9. A method of manufacturing a cholinergic/serotoninergic chimeric receptor comprising transforming a host cell with the vector of claim 3.

10. A method of screening for compounds that bind to a region of the cholinergic/serotoninergic chimeric receptor of claim 7 selected from the N-terminal domain, C-terminal domain, and the extracellular loop between TM2-TM3, to modulate the activity of a neuronal nicotinic receptor.

11. The method of claim 10, wherein the screening is assessed by binding or activity-based assays and determining whether the test compound binds or modulates the chimeric receptor, wherein the binding or modulation is indicative that the test compound binds or modulates the neuronal nicotinic receptor.

12. A method of screening for a compound that binds or modulates the activity of a neuronal nicotinic receptor, comprising:
introducing a host cell expressing the cholinergic/serotoninergic chimeric receptor of claim 7 into an acceptable medium; and
monitoring an effect in said host cell indicative of binding or modulation of the test compound with the cholinergic/serotoninergic chimeric receptor, wherein the binding or modulation is indicative that the test compound binds or modulates the neuronal nicotinic receptor.

13. A kit comprising a host cell transformed or transfected with an expression vector comprising the recombinant nucleic acid of claim 1.
